(19) Europäisches Patentamt
European Patent Office
Office européen des brevets

(11) Publication number: **0 315 118 A2**

(12) # EUROPEAN PATENT APPLICATION

(21) Application number: 88118171.3

(22) Date of filing: 01.11.88

(51) Int. Cl.4: C12N 15/00 , C07K 7/10 , C12N 1/20 , C12N 1/18 , C12P 21/02 , A61K 37/02

The title of the invention has been amended (Guidelines for Examination in the EPO, A-III, 7.3).

(30) Priority: 02.11.87 JP 275613/87
12.12.87 JP 313155/87
17.06.88 JP 148158/88

(43) Date of publication of application: 10.05.89 Bulletin 89/19

(84) Designated Contracting States: AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Masaki, Tomoh
210-1, 1267 Takezono 3-chome
Tsukuba-shi Ibaraki 305(JP)
Inventor: Yanagisawa, Masashi
907-303, 8-14, Takezono 1-chome
Tsukuba-shi Ibaraki 305(JP)
Inventor: Kurihara, Hiroki
3-6-904, Kameido 3-chome
Koto-ku Tokyo 136(JP)
Inventor: Onda, Haruo
201, 17-1, Namiki 3-chome
Tsukuba-shi Ibaraki 305(JP)
Inventor: Itoh, Yasuaki
202, 17-1, Namiki 3-chome
Tsukuba-shi Ibaraki 305(JP)
Inventor: Fujino, Masahiko
10-7, Hibarigaoka 2-chome
Takarazuka-shi Hyogo 665(JP)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

(54) **DNA coding for endothelin and use thereof.**

(57) (1) a DNA sequence containing a DNA segment coding for porcine or human endothelin, (2) a precursor protein of porcine or human endothelin, (3) a transformant bearing a DNA sequence containing a DNA segment coding for percine or human endothelin, and (4) a method for preparing mature porcine or human endothelin or a porcine or human endothelin precursor are disclosed.

The cells gene-infected or transformed with the DNA of the present invention can allow a large amount of endothelin to be produced, even in various cells in which little or no endothelin is originally produced. Therefore, the production of endothelin can be advantageously achieved.

Endothelin can be utilized as hypotension therapeutic agents or local vasoconstrictors to animals including humans.

EP 0 315 118 A2

# DNA AND USE THEREOF

## BACKGROUND OF THE INVENTION

The present invention relates to a DNA sequence containing a DNA segment coding for a vasoconstrictive peptide, namely endothelin, a precursor protein of endothelin, a DNA sequence containing a DNA segment coding for the endothelin precursor protein, a transformant containing the above DNA sequence and a method for preparing the precursor protein and a mature peptide (endothelin), as well as to pharmaceutical compositions employing such polypeptides. The above-described endothelin includes porcine endothelin and human endothelin. In this specification, the precursor protein is preferably used to describe a protein which includes an amino acid sequence of a mature peptide (endothelin) and has a portion or all of an amino acid sequence coded with endothelin cDNA at the N-terminus or both termini thereof.

There have been reports of endothelium-dependent vasoconstrictor reactions to various mechanical and chemical stimuli as well as endothelium-dependent vasodilative reactions. For example, it is known that vasoconstrictions can be induced by a mechanical load such as vascular stretch or increased vascular inner pressure, or can be chemically induced by such agents as thrombin, noradrenaline, vasopressin, bradykinin, conditions of anoxia, etc. It has also been reported that noradrenaline-induced vasoconstriction can be enhanced by use of neuropeptide Y. [K. Takemoto, Proc. Natl. Acad. Sci. U.S.A. 79, 5485 (1982); C. Minth et al.,ibid. 81, 4577 (1984)]. However, no endothelium-derived humoral factor mediating these constrictor reactions has yet been identified. Endothelial cell-derived coronary vascular constrictor factors (each having molecular weights of 8,500 and 3,000) are described in Am. J. Physiol. 248, C550 (1985) A. Kristine et al., and J. Cell. Physiol. 132, 263 (1987)(R. F. O'Brien et al.,). However, their structures are unknown. An endothelial cell-derived peptide-like substance is also described in J. Pharmac. Exp. Ther. 236, 339 (1985) (M. N. Gillespie et al.,). However, the structure of that substance is also unknown.

Certain peptides having a vasoconstrictor activity are known, such as vasopressin, bradykinin and the like, the amino acid sequences of which have been determined. There have been no reports, however, that such peptides were obtained from mammalian or bird vascular endothelial cells. Although, there is a report that angiotensin having a vasoconstrictor activity is obtained from endothelial cells of bovine aortas [I. Kifor and V. J. Dzay, Circ. Res. 60, 422 (1987)], angiotensin is a peptide having a molecular weight of only about 1,000.

The present inventors have discovered a novel peptide which has a strong vascular smooth muscle constrictor activity which is obtained from mammalian or bird vascular endothelial cells and has a molecular weight of 2,500 ± 300, and have filed a Japanese Patent Application (J. P. Application No. 255381/87). The present inventors et al. have named this novel peptide "Endothelin".

Endothelin has 21 amino acid residues, including four cystein groups located at the 1st, 3rd, 11th and 15th residue from the N-terminus of the amino acid sequence, which form two sets of disulfide bonds. One of the combinations of the disulfide bonds may be 1-15 and 3-11 cysteins and the other combination may be 1-11 and 3-15. Activity of the former combination is higher than the latter one, and that combination is thus preferred.

One of the isolated and purified peptides from porcine vascular endothelial cells is elucidated to be a peptide consisting of the following 21 amino acid residues (hereinafter referred to as endothelin A) from amino acid analysis (ninhydrin method), molecular weight measurement and other data;

Cys Ser Cys Ser Ser Leu Met Asp Lys Glu Cys Val Tyr Phe

Cys His Leu Asp Ile Ile Trp

and has 2 sets of S-S bonds between Cys and Cys. The molecular weight thereof is 2,492.

As a precursor of the above endothelin A from porcine aortas, a peptide comprised of 203 amino acid residues (hereinafter referred to as a porcine endothelin precursor), was also obtained. Amino acid sequence of the endothelin is shown in Fig. 2. The framed portion corresponds to mature porcine endothelin.

Previously, the peptides described above were obtained only by the method wherein the vascular endothelial cells of a mammal or a bird were incubated, followed by isolation and purification from the culture. It would be preferable to have a simpler method where the peptide can be obtained in large amounts.

## BRIEF DESCRIPTION OF THE DRAWINGS

Fig. 1 is a simplified restriction enzyme map of porcine endothelin cDNA;

Fig. 2 shows a nucleotide sequence of porcine endothelin cDNA and an amino acid sequence of porcine endothelin presumed therefrom.

Fig. 3 is a simplified restriction enzyme map of the human endothelin precursor or the mature peptide cDNA;

Fig. 4 shows a nucleotide sequence of the human endothelin precursor or the mature peptide cDNA and an amino acid sequence of the human endothelin precursor or the mature peptide cDNA presumed therefrom;

Fig. 5 is a scheme of the construction of a human endothelin expression vector whose host is yeast; and

Fig. 6 is a comparison of nucleotide and deduced amino acid sequences of human and porcine endothelin precursor.

Fig. 7 is a scheme of the construction of a plasmid for expression of COOH-mature endothelin.

Fig. 8 is a scheme of the construction of a plasmid pTS 4007.

Fig. 9 shows the western blotting analysis of the expression product of the E. coli N4830/pTS 4007.

Fig. 10 is a scheme of the construction of a plasmid pTS 6003.

Fig. 11 is a graph showing human endothelin production of COS 7 transfected with pTS 6003.

## SUMMARY OF THE INVENTION

The present inventors have discovered a method for preparing a large amount of the foregoing novel peptide (endothelin) having a vasoconstrictor action, which comprises cloning complementary DNA of endothelial cell endothelin of porcine aortas, and identified the whole nucleotide sequence thereof, and have succeeded in pioneering mass production of endothelin by gene recombination. The nucleotide sequence of the porcine endothelin is shown in Fig. 2.

Further, the present inventors synthesized chemically a DNA segment coding for a part of the peptide of porcine endothelin and succeeded for the first time in cloning cDNA coding for human endothelin from a human placenta-derived cDNA library by using the synthesized DNA segment as a probe. Further, the present inventors identified the whole nucleotide sequence of the cDNA, defined the amino acid sequence (Fig. 4) of human endothelin and the precursor protein thereof, and succeeded in pioneering mass producion of these by gene recombination.

Mature human endothelin is identical to the above porcine endothelin A, and corresponds to Nos. 53 to 73 amino acids, Cys Ser Cys Ser Ser Leu Met Asp Lys Glu Cys Val Tyr Phe Cys His Leu Asp Ile Ile Trp, in Fig. 4. The molecular weight was calculated as 2,492.

However, the precursor protein of human endothelin consists of 212 amino acid residues (Fig. 4) and has more amino acid residues than the precursor of porcine endothelin by 9 residues. The difference is also observed in amino acid sequence as shown in Fig. 6. In Fig. 6, H stands for human endothelin and P stands for porcine endothelin.

The endothelin of the present invention may modified by, for example, the addition, deletion or substitution of amino acid residues as long as the vasoconstrictive property is not lost. The DNA sequence of the present invention comprises a sufficient number of the nucleotides shown in Figure 2 or 4 to produce a peptide whose mature product has vasoconstrictive activity.

In accordance with the present invention, there are provided (1) a DNA sequence containing a DNA segment coding for endothelin, preferably porcine or human endothelin, (2) a precursor protein of endothelin, preferably porcine or human endothelin, (3) a transformant bearing a DNA sequence containing a DNA segment coding for endothelin, preferably porcine or human endothelin, and (4) a method for preparing mature porcine or human endothelin or a porcine or human endothelin precursor which comprises incubating a transformant described in (3), producing and accumulating a protein in a culture, and collecting the protein thus obtained.

As the DNA sequence of the present invention coding for porcine endothelin, any DNA may be used so long as DNA contains a nucleotide sequence coding for the amino acid sequence of the desired porcine endothelin peptide, such as mature porcine endothelin. For example, DNA containing the nucleotide sequence shown in Fig. 2 or DNA containing a portion thereof is preferably used.

As the DNA sequence coding for human endothelin, any DNA may be used so long as DNA contains a nucleotide sequence coding for the amino acid sequence of the desired human endothelin peptide, such as mature human endothelin. For example, DNA containing the nucleotide sequence shown in Fig. 4 or DNA containing a portion thereof is preferably used.

In the present invention, for example, an expression vector having the DNA sequence containing the nucleotide sequence coding for endothelin can be prepared by the following process:

(a) RNA coding for endothelin is isolated.

(b) Single stranded complementary DNA (cDNA) is synthesized from the RNA, followed by synthesis of double stranded DNA.

(c) The complementary DNA is introduced in a cloning vector such as a plasmid or a phage.

(d) A host is transformed with the recombinant DNA thus obtained.

(e) After culturing of the transformant thus obtained, the plasmid or the phage containing the desired DNA is isolated from the transformant by an appropriate method such as hybridization with a DNA probe coding for a portion of endothelin or immunoassay using anti-endothelin antibody.

(f) The desired cloned DNA sequence is cut out from the recombinant DNA.

(g) The cloned DNA sequence is ligated downstream from a promoter in the expression vector.

The RNA coding for endothelin can be obtained from various endothelin-producing cells, for example, endothelial cells of porcine or human aortas, human placenta etc.

The method for preparing RNA from the endothelin-producing cells includes the guanidine thiocyanate method [J. M. Chirgwin et al., Bio-chemistry, 18, 5294 (1979)] and the like.

Using the RNA thus obtained as a template, cDNA is synthesized by use of reverse transcriptase, for example, in accordance with the method of H. Okayama et al. [Molecular and Cellular Biology, 2, 161 (1979); ibid., 3, 280 (1983)]. The cDNA thus obtained is introduced in the plasmid.

The plasmid in which the cDNA is introduced includes, for example, pBR322 [Gene, 2, 95 (1977)], pBR325 [Gene, 4, 121 (1978)], pUC12 [Gene, 19, 259 (1982)], and pUC13 [Gene, 19, 259 (1982)], each derived from Escherichia coli, and pUB110 derived from Bacillus subtilis [Biochemical and Biophysical Research Communication, 112, 678 (1983)]. However, any other plasmid can be used as long as it is replicable and growable in the host. The phage vector in which the cDNA is introduced includes, for example, λgt11 [R. Young and R. Davis, Proc. Natl. Acad. Sci., U.S.A., 80, 1194 (1983)] and the like. However, any other phage vector is available, if growable in the host.

The method for introducing the cDNA in the plasmid includes, for example, the method described in T. Maniatis et al., Molecular Cloning, Cold Spring Laboratory, p.239 (1982). The method for introducing the cDNA in the phage vector includes, for example, the method of T. V. Hyunh et al. [DNA Cloning, A Practical Approch, 1, 49 (1985)]. The plasmid thus obtained is introduced in the appropriate host cell such as Escherichia and Bacillus.

Examples of Escherichia described above include Escherichia coli K12DH1 [Proc. Nat]. Acad. Sci. U.S.A., 60, 160 (1968)], M103 [Nucleic Acids Research, 9, 309 (1981)], JA221 [Journal of Molecular Biology, 120, 517, (1978)], HB101 [Journal of Molecular Biology, 41, 459 (1969)], C600 [Genetics, 39, 440 (1954)] and the like.

Example of Bacillus described above, includes Bacillus subtilis MI114 [Gene, 24, 255(1983)], 207-21 [Journal of Biochemistry, 95, 87(1984)] and the like.

The method for transforming the host with the plasmid includes, for example, the calcium chloride method or the calcium chloride/rubidium chloride method described in T. Maniatis et al., Molecular Cloning, Cold Spring Harbor Laboratory, p.249 (1982).

When phage vector is used, for example, the phage vector can be transduced into multiplied Escherichia coli, using the in vitro packaging method.

The desired clone is selected from the transformant thus obtained by a known method such as the colony hybridization method [Gene, 10, 63 (1980)] and the DNA base sequence determination method [Proc. Natl. Acad. Sci. U.S.A., 74, 560 (1977); Nucleic Acids Research, 9, 309 (1981)].

Thus, a microorganism bearing the vector which has the DNA sequence containing the nucleotide sequence coding for the cloned endothelin is obtained.

Then, the plasmid or the phage vector is isolated from the microorganism.

The isolating method includes the alkali method [H. C. Birmboim et al., Nucleic Acids Research, 1, 1513 (1979)] and the like.

The foregoing plasmid or phage vector which has the DNA sequence containing the nucleotide sequence coding for the cloned endothelin is used as it is, or cut out with a restriction enzyme if desired.

The gene cloned is linked downstream from the promoter in the vehicle (vector) suitable for expression, whereby the expression vector can be obtained.

The human cDNA libraries containing human endothelin cDNA can be obtained by numerous techniques well known in the art including purchasing it from the market, though obtainable by the methods described above. For example, the cDNA library of human placentas is available from Clontech Laboratories, Inc., U.S.A.

The method for cloning the human endothelin cDNA from the human cDNA library includes, for example, the method of Hyunh et al using phage vector λgt11 and anti-porcine endothelin antibody [DNA Cloning, A Practical Approach, p.49 (1985)] and the colony hybridization or plaque hybridization method using oligonucleotide chemically synthesized on the basis of the amino acid sequence of porcine endothelin as a probe [T. Maniatis et al., Molecular Cloning, Cold Spring Harbor Laboratory (1982)].

The human endothlin cDNA thus cloned is subcloned to, for example, pBR322, pUC12, pUC13, pUC18, pUC19, pUC118, pUC119 or the like to obtain the human endothelin cDNA, if necessary.

The nucleotide sequence of the DNA sequence thus obtained is determined by, for example, the Maxam-Gilbert method [A. M. Maxam and W. Gilbert, Proc. Natl. Acad. Sci. U.S.A. 74, 560 (1977)] or the dideoxy method [J. Messing et al., Nucleic Acids Research 9, 309 (1981)], and the existence of the human endothelin cDNA is confirmed in comparison with the known amino acid sequence.

As described above, the DNA (human endothelin cDNA) (Fig. 4) coding for the precursor protein of human endothelin is obtained.

The restriction enzyme fragment map of the DNA sequence coding for the precursor protein of human endothelin obtained in Example 2 which will hereinafter be described is shown in Fig. 3. The nucleotide sequence of the cDNA as determined by the dideoxy method and the amino acid sequence ascertained from the nucleotide sequence are shown in Fig. 4.

The DNA sequence coding for the precursor protein of human endothelin cloned as described above can be used as it is, or cut out by digestion with a restriction enzyme if desired, according to the intended use.

The region intended to be expressed is cut out from the cloned DNA and ligated downstream from the promoter in a vehicle (vector) suitable for expression, whereby the expression vector can be obtained.

The DNA sequence has ATG as a translation initiating codon at the 5′-terminus thereof and may have TAA, TGA or TAG as a translation terminating codon at the 3′-terminus. These translation initiating codon and translation terminating codon may be added by use of an appropriate synthetic DNA adaptor. Further, in order to express the DNA sequence a promoter is ligated to the upstream thereof.

The vector includes the foregoing plasmids derived from Escherichia coli such as pBR322, pBR325, pUC12 and pUC13, the plasmids derived from Bacillus subtilis such as pUB110, pTP5 and pC194, plasmids derived from yeast such as pSH19 and pSH15, bacteriophage such as λ phage, and animal viruses such as retroviruses and vaccinia viruses.

As the promoter used in the present invention, any promoter is available so long as the promoter is suitable for expression corresponding to the host cell used for the gene expression.

When the host cell used for transformation is Escherichia, it is preferable that a trp promoter, a lac promoter, a recA promoter, a λPL promoter, a 1pp promoter or the like are used. When the host is Bacillus, it is preferable that a SPO1 promoter, a SPO2 promoter, a penP promoter or the like. When the host is yeast, it is preferred that a PHO5 promoter, a PGK promoter, a GAP promoter, an ADH promoter or the like are used. Particularly, it is preferable that the host is Escherichia and the promoter is the trp promoter or the λPL promoter.

When the host is an animal cell, a SV40-derived promoter, a retrovirus promoter etc. are preferably used. Particularly, the SV40-derived promoter is preferable.

By using a vector containing the DNA sequence coding for the endothelin thus constructed, the transformant is prepared.

The host cells include, for example, Escherichia, Bacillus, yeast and animal cells.

As examples of Escherichia and Bacillus described above, there can be mentioned the similar strains described above.

Examples of the yeast described above include, for example, Saccharomyces cerevisiae AH22, AH22R$^-$, NA87- 11A, and DKD-5D.

Examples of the animal cell include, for example, monkey cell COS-7, Vero, Chinese hamster cell CHO, mouse L cell and human FL cell.

The transformation of Escherichia described above is carried out, for example, according to the method described in Proc. Natl. Acad. Sci. U.S.A., 69, 2110 (1972), Gene, 17, 107 (1982) or the like.

The transformation of Bacillus is conducted, for example, according to the method described in Molecular & General Genetics, 168, 111 (1979) or the like.

The transformation of the yeast is carried out according to, for example, the method described in Proc. Natl. Acad. Sci. U.S.A., 75, 1929 (1978).

The transformation of the animal cell is carried out according to, for example, the method described in Virology, 52, 456 (1973).

Thus, a transformant transformed with the vector containing the DNA sequence coding for endothelin is obtained.

When the transformant wherein the host is Escherichia or Bacillus is cultured, a liquid medium is particularly suitable as a medium used for culture. Carbon sources, nitrogen sources, inorganic compounds and others necessary for growth of the transformant are contained therein. The carbon source includes, for example, glucose, dextrin, soluble starch and sucrose. The nitrogen source includes inorganic or organic materials such as ammonium salts, nitrates, corn steep liquor, peptone, casein, meat extracts, soybean meal and potato extract solution. The inorganic compound includes, for example, calcium chloride, sodium dihydrogenphosphate and magnesium chloride. Further, yeasts, vitamins, growth promoting factors and so on may be added thereto.

The pH of the medium is preferably about 5 to 8.

As the medium used for culturing of Escherichia, there is preferred, for example, M9 medium containing glucose and Casamino Acids (Miller, Journal of Experiments in Molecular Genetics, 431-433, Cold Spring Harbor Laboratory, New York, 1972). In order to make the promoter act efficiently, the drug such as 3-indolylacrylic acid may be added thereto if necessary.

When the host is Escherichia, the culturing is usually carried out at about 15 to 43° C for about 3 to 24 hours, with aeration or agitation if necessary.

When the host is Bacillus, the culturing is usually carried out at about 30 to 40° C for about 6 to 24 hours, with aeration or agitation if necessary.

When the transformant wherein the host is yeast is cultured, there is used, for example, Burkholder minimum medium [K. L. Bostian et al., Proc. Natl. Acad. Sci. U.S.A., 77, 4505 (1980)] as the medium. The pH of the medium is preferably adjusted to about 5 to 8. The culturing is usually carried out at about 20 to 35° C for about 24 to 72 hours, with aeration or agitation if necessary.

When the transformant wherein the host is the animal cell is cultured, there can be mentioned as the medium, for example, MEM medium containing about 5 to 20% fetal calf serum [Science, 122, 501 (1952)], DMEM medium [Virology, 8, 396 (1959)], RPMI1640 medium [Journal of the American Medical Association, 199, 519 (1967)] and 199 medium [Proceeding of the Society for the Biological Medicine, 73, 1 (1950)]. The pH is preferably about 6 to 8. The culturing is usually carried out at about 30 to 40° C for about 15 to 60 hours, with aeration or agitation if necessary.

Mature endothelin or precursor endothelin can be isolated and purified from the culture described above, for example, by the following method.

When the precursor protein of endothelin or the mature peptide (endothelin) is extracted from the cultured cells, the cells are collected by a known method after the culturing. Then, the collected cells are suspended in an appropriate buffer solution and disrupted by ultrasonic treatment, lysozyme and/or freeze-thawing. Thereafter, a crude extracted solution of the precursor protein of endothelin or the mature peptide is obtained by centrifugation or filtration. The buffer solution may contain a protein denaturant such as urea or guanidine hydrochloride, or a surface-active agent such as Triton-100.

When the precursor protein of endothelin or the mature peptide is secreted in the culture solution, the supernatant is separated from the cells by a known method after the conclusion of incubation, and then collected.

The separation and purification of the precursor protein of endothelin or the mature peptide contained in the culture supernatant or the extracted solution thus obtained can be performed by an appropriate combination of known separating and purifying methods. Known separating and purifying methods include utilizing solubility such as salting out and solvent precipitation, methods mainly utilizing a difference in molecular weight such as dialysis, ultrafiltration, gel filtration and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse phase high performance liquid chromatography and methods utilizing a difference in isoelectric point such as isoelectric point electrophoresis.

The activity of endothelin precursor or the mature peptide thus formed can be measured by the enzyme immunoassay using a specific antibody, protein phosphorylation method etc. If the products has vasoconstrictive activity, the activity may be measured as an index.

The cells gene-infected or transformed with the DNA of the present invention can allow a large amount of endothelin to be produced, even in various cells in which little or no endothelin is originally produced. Therefore, the production of endothelin can be advantageously achieved.

Endothelin not only can be utilized as hypotension thereapeutic agents or local vasoconstrictors but also give a clue to analysis of the mechanism of the vasoconstrictor reactions in vivo or to elucidation of the antagonists to the vasoconstrictor factors.

For example, endothelin has such an effect as preventing various hemorrhage, for example, gastric or esophageal hemorrhage as vasoconstrictors. And the peptides have also an effect to curing various shock symptoms. The peptides can be administered orally, locally, intraveneously or parenterally, preferably locally or intravenously. The dose is 0.001 µg - 100 µg/kg, preferably 0.01 µg - 10 µg/kg. The dose is preferably dependent on weight and preferably is in the form of a solution in 1 - 10 ml of isotonic sodium chloride solution.

Endothelin can be formed into various preparations, containing endothelin and additional components, such as emulsion, hydrated mixture, tablet, solution, powder, granules, capsule, pill etc. Said additional components include pharmaceutically acceptable vehicles, disintegrators, lubricants, binders, dispersants, plastisizers, fillers, carriers etc. As examples of the additional components, the examples of vehicles are lactose, glucose and white sugar; those of disintegrators are starch, sodium alginate, agar powder and carboxymethyl cellulose calcium; those of lubricants are magnesium stearate, talc and liquid paraffin; those of binders are syrup, gelatin solution, ethanol and polyvinyl alcohol; those of dispersants are methyl cellulose, ethyl cellulose and shellac; and those of plasticizers are glycerin and starch.

There were hereinbefore described in detail the cloning of endothelin the preparation of the transformant, the production of endothelin by use of the transformant and the utility thereof.

When bases, amino acids and so on are indicated by the abbreviations in this specification and drawings, the abbreviations adopted by IUPAC-IUB Commission on Biochemical Nomenclature or commonly used in the art are employed. For example, the following abbreviations are used. When the optical isomer is capable of existing with respect to the amino acids, the L-form is represented unless otherwise specified. DNA : Deoxyribonucleic acid

cDNA : Complementary deoxyribonucleic acid
A : Adenine
T : Thymine
G : Guanine
C : Cytosine
RNA : Ribonucleic acid
dATP : Deoxyadenosine triphosphate
dTTP : Deoxythymidine triphosphate
dGTP : Deoxyguanosine triphosphate
dCTP : Deoxycytidine triphosphate
ATP : Adenosine triphosphate
EDTA : Ethylenediaminetetraacetic acid
SDS : Sodium dodecyl sulfate
Gly or G : Glycine
Ala or A : Alanine
Val or V : Valine
Leu or L : Leucine
Ile or I : Isoleucine
Ser or S : Serine
Thr or T : Threonine
Cys or C : Cysteine
Met or M : Methionine
Glu or E : Glutamic acid
Asp or D : Aspartic acid
Lys or K : Lysine
Arg or R : Arginine
His or H : Histidine
Phe or F : Phenylalanine
Tyr or Y : Tyrosine
Trp or W : Tryptophan
Pro or P : Proline

Asn or N : Asparagine
Gln or Q : Glutamine

With respect to endothelin of the present invention, a portion of the amino acid sequence may be modified, namely there may be addition, elimination or replacing with other amino acid as long as the vasoconstrictive property is not lost.

The present invention will hereinafter be described in detail with the following Examples. It is understood of course that these Examples are not intended to limit the scope of the invention.

Transformant Escherichia coli XL-1/ppET4.4 obtained in Example 3 described later is deposited in Fermentation Research Institute, Agency of Industrial Science and Technology, Ministry of International Trade and Industry, Japan (FRI) with the accession number FERM P-9683 on October 30, 1987. This microorganism is also deposited in the Institute for Fermentation, Osaka, Japan (IFO) with the accession number IFO 14670 on November 2, 1987.

Transformant Escherichia coli JM103/pHET4-3 obtained in Example 6 described later was deposited in Fermentation Research Institute, Agency of Industrial Science and Technology, Ministry of International Trade and Industry, Japan (FRI) with the accession number FERM P-9755 on December 10, 1987. This microorganism was also deposited in the Institute for Fermentation, Osaka, Japan (IFO) with the accession number IFO 14675 on December 4, 1987.

Transformant Saccharomyces cerevisiae AH22R⁻/pGLD906-20 was similarly deposited in the Institute for Fermentation, Osaka, Japan (IFO) with the accession number IFO 10435 on June 16, 1988. This microorganism was deposited in Fermentation Research Institute, Agency of Industrial Science and Technology, Ministry of International Trade and Industry, Japan (FRI) with the accession number FERM BP-1925 on June 24, 1988.

## Reference Example

(1) Assay of Vascular Smooth Muscle Constrictor Activity

Porcine right coronary artery spiral specimens (2 X 20 mm) with the intima denuded by rubbing with a small swab are suspended in 3 ml of Krebs-Ringer solution maintained at 37° C and saturated with a mixed gas containing 5% carbon dioxide and 95% oxygen by volume. After setting the basal tension to 1 g, the isometric tension is measured with tension transducers.

(2) Assay of Cardiotonic Action

Instead of the porcine right coronary artery spiral specimens used in the assay described in the above item (1), guinea pig right atrium suspended specimens are used, and the tension and the heart rate per minute are measured according to the same procedure as described in (1).

(3) $ED_{50}$

Median effective dose. Effective dose for 50% of tested animals.

## Example 1

Preparation of cDNA from Endothelial Cells of Porcine Aortas.

Endotherial cells of porcine aortas were monolayered cultured in Eagle's minimal essential medium containing 10% fetal calf serum. RNA was extracted from $10^8$ cells thus cultured by the guanidine-hot phenol method (T. Maniatis et al., Molecular cloning-A laboratory manual, p.194-195, 1982). The RNA thus extracted was purified by oligo(dT) cellulose column chromatography to provide poly(A) RNA (ibid. p.197-198). Using the poly(A) RNA as a template, a cDNA library was synthesized by the method of C. J. Watson and J. F. Jacson (D. M. Glover, DNA cloning, vol. I, p.79-88, 1985). Then, the cDNA was cloned into the EcoRI site of phage vector λgt10 according to the method of T. V. Huynh et al. (ibid. p.49-78) to prepare a cDNA library which consisted of 2 X $10^6$ independent clones.

## Example 2

### Preparation of DNA Probe Coding for Part of Endothelin

Of the DNA sequences coding for the amino acid sequence composed of the 7th to the 20th residues of endothelin, Met-Asp-Lys-Glu-Cys-Val-Tyr-Phe-Cys-His-Leu-Asp-Ile-Ile, a codon having the highest usage frequency was selected according to the method of R. Lathe [J. Mol. Biol., 183, 1-12 (1985)], and a DNA probe having the following sequence was synthesized.

$^{5'}$ ATGGACAAGGAGTGTGTCTACTTCTGCCATCTGGACATCATC$^{3'}$

The 5′-terminus of the DNA probe was phosphorylated with $^{32}P$ by using polynucleotide kinase. The phosphorylated DNA probe was used for screening the cDNA library.

## Example 3

### Isolation of Endothelin cDNA and Determination of Nucleotide Sequence Thereof

Escherichia coli C600hf1 was infected with the foregoing gt10 cDNA library and plated. And then phage plaques appeared. A portion of plaque DNA was transferred to a nylon film and hybridized with the DNA probe labeled with $^{32}p$ in Example 2. The hybridization was carried out in the presence of 20% formaldehyde at 42°C. Each of 38 clones positive to hybridization was isolated. Then, a cDNA portion of λpET4 which was one of the clones described above was cut out with EcoRI and recloned into the EcoRI site of plasmid pUC118 to prepare plasmid ppET4.4. By transforming Escherichia coli XL-1 with this plasmid, transformant Escherichia coli XL-1/ppET4.4 was obtained. The cDNA portion included in this plasmid was 1.8 Kbp, and the simplified restriction enzyme map thereof is shown in Fig. 1. In the figure, ▭ shows a porcine endothelin precursor code region, ▬ shows a mature porcine endothelin region, ⬚ shows a signal peptide region (presumed), ▲ shows a basic residue pair, ⋀⋀⋀ shows poly(A) and → shows the direction and length of sequencing. The nucleotide sequence of the cDNA portion was determined by the method of Sanger [Proc. Natl. Acad. Sci. U.S.A. 74, 5463-5467 (1977)]. The nucleotide sequence and the amino acid sequence of porcine endothelin precursor presumed thereform are shown in Fig. 2. The region surrounded by ▭ ; shows mature endothelin.

## Example 4

### (1) Synthesis of Endothelin

Endothelin A was synthesized by conventional methods, using 0.7 g (0.5 m mole) of a commercial available protected tryptophan resin [Boc-Trp(CHO)-PAM resin, Applied Biosystems Inc., U.S.A.] and using a peptide synthesizer (Model 430A, Applied Biosystems Inc., U.S.A.).

Condensation process was conducted as follows:

The resin was treated with 50% trifluoroacetic acid in methylene chloride and a protected terminal amino group by Boc group was deprotected to free amino group. To the free amino group, the following protected amino acids were condensed in turn according to the amino acid sequence of Endothelin (Compound I) from the C-terminal in the presence of dicyclohexyl carbodiimide (DCC);

Boc-Ile, Boc-Asp(OBzl), Boc-Leu, Boc-His(Tos), Boc-Cys(Acm), Boc-Tyr(Br-z), Boc-Val, Boc-Phe, Boc-Glu-(OBzl), Boc-Lys(Cl-Z), Boc-Met and Boc-Ser(Bzl).

800 mg of 1.8 g of the protected endothelin resin thus obtained was swelled with 1 ml of anithole and 1 ml of 1,2-ethanedithiole and then treated with 10 ml of hydrogen fluoride at 0°C for 60 minutes followed by removal of excess hydrogen fluoride under reduced pressure. After washing the residue with 5 ml of ethyl acetate, it was extracted with 50% aqueous acetic acid and applied on a dextran gel column (Sephadex LH-20) (2 x 90 cm). Main active fractions eluted with the above solvent were collected to be lyophylized and then 120 mg of white powder was obtained. Twenty (20) mg of the powder was dissolved in 20 ml of 80% aqueous acetic acid and 15 mg of trifluoroacetate-mercury(II) was added thereto. After stirring at room temperature for 60 minutes, it was diluted with 30 ml of the above solvent and hydrogen sulfide gas was let therein and a deposit was filtered off and lyophilized. It was dissolved into 400 ml of dilute acetic acid and adjusted at pH 8 with ammonium hydrogen carbonate. After it was subjected to air-oxidation for 6 hours, acetic acid was added thereto to adjust it to pH 3 and it was lyophilized. It was applied on a Sephadex LH-20 column (2x90 cm) filled with 30% acetic acid and main fractions were collected. Further, it was fractionated by using HPLC (Column: YMC, Solvent: eluted by a linear gradient of 0.1% trifluoroacetic acid and acetonitrile containing 0.1% trifluoroacetic acid) and 2.7 mg of the desired product was obtained.

The synthesized endothelin A was eluted by HPLC at the same position as that of the natural extracted endothelin:

Assay conditions (1)

Column: Nucleosile 50 DS-H(Chemco) (4.6 mm ø x 250 mm)
Eluent:

A (0.1% aqueous trifluoroacetic acid),
B (50% aqueous acetonitrile containing 0.1% trifluoroacetic acid).

A linear gradient elution from the eluent A to the eluent B for 20 minutes.
Flow rate: 1.0 ml/minute.

Assay conditions (2)

Column: DEAE-2SW(Toyo Soda) (4.6 mm ø x 250 mm)
Eluent:

A (10 mM Tris.HCl pH 7.5),
B (A containing 1 M NaCl).

A linear gradient elution from the eluent A to the eluent B for 40 minutes.
Flow rate: 1.0 ml/minute.

Elution position:
21.5 minutes [assay conditions (1)]
21.3 minutes [assay conditions (2)]

(2) Assay

The activity of endothelin A obtained in (1) above was assayed by the method of Reference Example (1) and (2).

$ED_{50}$ by the assay (1) for smooth muscle constrictor activity to porcine coronary artery was 4 to $5 \times 10^{-10}$ mole/l.

$ED_{50}$ by the assay (2) for cardiotonic action to guinea pig right atrium was $1 \times 10^{-9}$ mole/l.

(3) Injection preparation

12 μg of endothelin A obtained in (1) is dissolved in isotonic sodium chloride solution for injection, then filtered by a Millipore filter, and lyophilized. An intravenous injection preparation is prepared, for use, by dissolving the lyophilized product in isotonic sodium chloride solution for injection to a total volume of 5 ml.

## Example 5

Preparation of a DNA Probe Coding for a Portion of Porcine Endothelin

Presuming the messenger RNA sequence expected from the amino acid sequence composed of the 7th to the l6th residues of porcine endothelin,
Met-Asp-Lys-Glu-Cys-Val-Tyr-Phe-Cys-His, a DNA probe having the following sequence was chemically synthesized.

$^{5'}$TG GCA GAA GTA GAC GCA CTC CTT GTC CAT$^{3'}$

The 5′-terminus of this DNA probe was phosphorylated with $^{32}$p by using T4 polynucleotide kinase. The phosphorylated DNA probe was used for screening the cDNA library.

## Example 6

Isolation of Human Endothelin Precursor cDNA and Determination of the Nucleotide Sequence Thereof

Escherichin coli Y1090 was infected with the foregoing human placenta cDNA library (prepared by Clontech Laboratories, Inc.) and plated. And then phage plaques appeared. A portion of plaque DNA was transferred to a nitrocellulose film according to the report of W. Benton and R. Davis [Science 196, 180-182 (1977)] and hybridized with the DNA probe synthesized in Example 5 and labeled with $^{32}$p. The hybridization was carried out in the absence of formaldehyde at 55 °C. Each of 5 clones positive to hybridization was isolated. Then, a cDNA portion of HET4-3 which was one of the clones described above was cut out with EcoRI and recloned into the EcoRI site of plasmid pUC18 to prepare plasmid pHET4-3. By transforming Escherichia coli JM103 with this plasmid, transformant Escherichia coli JM103/pHET4-3 was obtained. The cDNA portion included in this plasmid was 1.2 Kbp, and the simplified restriction enzyme map thereof is shown in Fig. 3. In the figure, the empty box shows a region coding for a human endothelin precursor and the filled-in box shows a mature human endothelin region. The nucleotide sequence of the cDNA portion was determined by the method of Sanger [Proc. Natl. Acad. Sci. U.S.A. 74, 5463-5467 (1977)-]. The nucleotide sequence and the amino acid sequence of the human endothelin precursor presumed therefrom are shown in Fig. 4. The region surrounded by ▭ shows the mature peptide of human endothelin.

## Example 7

Construction of a Human Endothelin Expression Vector Whose Host Is Yeast and Transduction into Yeast

The plasmid pHET4-3 (10 µg) described in Example 6 was digested by restriction enzyme BglII and EcoRI (both Takara Syuzo Inc., Japan). Thereafter, a DNA fragment of 1.01 Kbp containing the code region of human endothelin was separated by agarose electrophoresis. To this DNA fragment (2 µg), 1 unit of DNA polymerase I Klenow fragment (Takara Syuzo Inc.) was added and reacted in a reaction solution (7 mM Tris-HCl, pH 7.5 / 20 mM NaCl / 7 mM $MgCl_2$ / 0.1 mM dATP, dGTP, dCTP, dTTP) at 37 °C for 1 hour to change the termini of the product to flush ends. Then, 0.1 µg of an Xho I linker, d(CCTCGAGG), (Takara Syuzo Inc.) was added thereto and reacted by use of 100 units of T4 DNA ligase (Takara Syuzo Inc.) in a reaction solution (66 mM Tris-HCl, pH 7.6 / 6.6 mM $MgCl_2$ / 10 mM dithiothreitol / 0.1 mM ATP) at 14 °C for 16 hours to link the Xho I linker. Further, 20 units of restriction enzyme Xho I (Nippon Gene Inc.) was added thereto and reacted at 37 °C for 3 hours to trim the DNA fragment. 0.5 µg of this DNA fragment was linked with 0.1 µg of a 9.4 Kbp DNA fragment obtained by cleaving expression vector PGLD906-1 for yeast (Japanese Patent Unexamined Publication No. 43991/1986) with restriction enzyme Sal I, by use of 20 units of T4 DNA ligase in the reaction solution described above to transform E. coli DH1 (Molecular Cloning, Cold Spring Harbor Laboratory, 1982). Plasmid PGLD906-20 was separated from the ampicillin-resistant transformant thus obtained (Fig. 5).

Using 3 µg of plasmid pGLD906-20, Saccharomyces cerevisiae AH22R$^-$ [Miyanohara et al., Proc. Natl. Acad. Sci. U.S.A. 80, 1 (1983)] was transformed by the protoplast method [Hinnen et al., Proc. Natl. Acad. Sci. U.S.A. 75, 1927 (1978)]. As a result, many strains of transformant AH22T$^-$/pGLD906-20 viable on a leucine-free medium were separated.

Example 8

Expression of Human Endothelin in a System Wherein Host Is Yeast

(i) Production of human endothelin

From many strains of transformant Saccharomyces cerevisiae AH22R⁻/pGLD906-20 obtained in Example 7, five strains were selected, and the human endothelin productivity thereof was assayed by the following method.

The medium of Kitano et al. [Bio/Technology 5, 281 (1987)] was dispensed in 5 ml portions into test tubes, and the transformant was inoculated thereto. Then, the shaking culture was carried out at 30°C for 3 days. Further, 1 ml of each culture was transferred into each of the test tubes which had the same medium dispensed in 10 ml portions, and the shaking culture was carried out at 30°C for 1 day. Subsequently, 3 ml of each culture thus obtained was transferred into each of 200 ml flasks containing 30 ml of the same medium as described above and incubated at 30°C for 2 days.

The obtained culture solutions were fractionated by centrifugation at 3,000 rpm for 10 minutes to separate into supernatants and cells. The cells were disrupted by the method of Rose et al. [Proc. Natl. Acad. Sci. U.S.A. 78, 2460 (1981)] to obtain extracted solutions. Namely, the cells collected from 10 ml of each culture solution were once washed with a SM buffer (85 mM NaCl, 1 mM $MgSO_4$, 20 mM Tris-HCl, pH 7.4) and then frozen at -80°C. To the frozen cells, 1 ml of a buffer for disruption (100 mM Tris-HCl, pH 8.0, 20% Glycerol, 1mM PMSF, 1 mM DTT) and 2 g of glass beads were added, and the cells were disrupted by vigorous agitation of the mixtures by a vortex mixer. The resultant products were centrifuged to obtain supernatants of cell extracted solutions.

The determination of endothelin was conducted by EIA based on the competitive assay. Peroxidase labeled (POD)-endothelin was used as a marker, and antiendothelin-rabbit polyclonal antibody was used as an antibody.

As a result, all of 5 strains of the transformant showed the production of human endothelin inside and outside the cells.

| Transformant | Human endothelin (ng/ml broth) | |
|---|---|---|
| | Supernatant | Cell extracted Solution |
| No.1 | 13.0 | 12.4 |
| 2 | 11.0 | 12.4 |
| 3 | 19.0 | 9.0 |
| 4 | 24.0 | 12.8 |
| 5 | 11.5 | 12.0 |

Example 9

Expression of COOH-matured Endothelin in Yeast

The plasmid pGLD 906-20 (Example 8) was digested with Bam HI and Hind III (1.66 kb) and this fragment was inserted into the Bam HI and Hind III site of M13 mp 18. Then site directed mutagenesis was conducted so that the amino acid residue No.22, valine, of endothelin was converted to stop codon TAA. This DNA fragment was inserted into the Bam HI and Hind III site of pGLD 906-20. The thus obtained plasmid was named pGLD 906-21 (Fig. 7) which expressed the COOH-matured endothelin in Saccharomyces cerevisiae. Transformant with this plasmid pGLD 906-21 was named Saccharomyces cerevisiae AH 22R⁻/pGLD906-21. This transformant produced COOH-matured endothelin 6 ng/ml in the culture medium and 25 ng/ml in the cells.

## Example 10

### Expression of Pre-pro Endothelin in E. coli

(i) Construction of a transformant

The plasmid pHET 40-3 was purified and digested with restrictin enzyme Bgl II and Eco RI. The thus obtained DNA fragment of 1 kb was inserted into the Eco RI, Bam HI sites of M13 mp 18. The nucleotide sequences in the endothelin cDNA, 5′TTTCAGAATGGAT was converted to 5′TTCCACCATGGAT by the method of site directed mutagenesis to make restriction enzyme site Nco I (Kunkel et al., Proc. Natl. Acad. Sci., U.S.A. 82, 488-492, 1985).

The thus obtained plasmid pTS 4001 was cleaved with Nco I, then teated with DNA polymerase, Klenow fragment, to have flat ends. The Eco RI linker 5′dGGAATTCC was connected to this DNA and digested with Eco RI. Thus, human pre-pro endothelin cDNA containing Eco RI site on both sides was obtained. This DNA fragment was inserted into the Eco RI site of pTB 281 which has the λpL promoter. The expression plasmid thus obtained was named pTS 4007 (Fig.8). Escherichia coli N4830 was trasformed with pTS 4007.

(ii) Expression of Pre-pro Endothelin

E. coli N4830/pTS 4007 obtained in Example 10 i) was inoculated into 2 ml of a liquid medium (pH 7.0) containing 1% Bacto tryptone (Difco Laboratories, USA), 0.5% Bacto yeast extract (Difco Laboratories, USA), 0.5% sodium chloride and 50 μg/ml Ampicilin in a 10 ml culture tube. After incubation at 30°C overnight on a swing rotor, the culture medium was transferred to a 250 ml flask containing 10 ml of M9 medium containing 0.5% casamino acids, 0.5% glucose and 50 μg/ml Ampicilin. Incubation was then conducted with shaking at 30°C for 4 hours and 42°C for further 2 hours. Cells were harvested from the culture broth by cenrifugation and treated with 7 M Guanidine-HCl in 0.1 M Tris-HCl, pH 8.0 at 0°C for 1 hour. The lysate was centrifuged for 15,000 rpm for 10 minutes and diluted with EIA buffer A. The calculated pre-pro endothelin produced in E. coli was 1 μg/ml culture broth.

The western blotting analysis of the cultured E. coli showed that the expression products with molecular weight about 25,000-28,000 were detected with anti-endothelin antibodies (Fig. 9).

## Example 11

### Expression of Endothelin in Animal Cell COS 7

The plasmid pHET 4-3 was cleaved with restriction enzyme Eco RI and the DNA fragment containing whole human endothelin cDNA was obtained. 2.5 μg of the thus obtained 1.17 kb DNA fragment was treated with T4 DNA polymerase to have flat ends and was connected with Bgl II linker dCAGATCTG by using T4 DNA ligase. The thus obtained DNA was digested with Bgl II to get Bgl II sites on each side of the DNA. This DNA fragment was inserted into the Bgl II sites of pTB 551 which had SV 40 promoter and polyadenylation signal site by using T4 DNA ligase. This ligated DNA preparation was used to transform E. coli DH 1 strain in the presence of Ampicillin (50 μg/ml) on 1.5% agarose plate containing LB broth. One of the clones which had plasmid with human pre-pro endothelin cDNA under the SV 40 promoter on right reading frame was selected and named pTS 6003 (Fig. 10).

The thus obtained plasmid pTB 6003, 5 μg was used to transfect the COS 7 cells previously seeded on 6 cm plastic dish in the culture medium Dulbecco's minimum essential medium (D-MEM) containing 10% fetal calf serum at 37°C in 5% $CO_2$ (Gorman et al., Science 221, 551-553, 1983). The culture medium of the cells were obtained after 24, 48 and 72 hours after DNA transfection and assayed with EIA specific for human endothelin. The COS 7 cells transfected with pTS 6003 synthesized the human endothelin as shown in Fig. 11.

The following microorganisms which are disclosed in the Examples and Reference Examples have been deposited in the Institute for Fermentation (IFO), Osaka, Japan and in the Fermentation Research Institute, Agency of Industrial Science and Technology (FRI), Japan. FERM BP is under the Budapest Treaty.

| Microorganism | IFO | FRI(FERM)(Deposit date) |
|---|---|---|
| Escherichia coli | | |
| 1. XL-1/ppET4.4<br>2. JM103/pHET4-3<br>3. N4830/pTS 4007<br>4. DHI/pTS6003 | 14670<br>14675 | P-9683(Oct. 30, 1987)<br>P-9755(Dec. 10, 1987)<br>BP-2130(Oct. 28, 1988)<br>BP-2131(Oct. 28, 1988) |
| Saccharomyces cerevisiae | | |
| 5. AH22R⁻/pGLD906-20<br>6. AH22R⁻/pGLD906-21 | 10435' | BP-1925(Jun. 24, 1988)<br>BP-2132(Oct. 28, 1988) |

## Claims

1. A DNA sequence which comprises a DNA segment coding for endothelin.
2. The DNA sequence according to claim 1 wherein the endothelin is porcine endothelin.
3. The DNA sequence according to claim 2, which has the DNA sequence shown in Fig. 2 or a portion thereof.
4. The DNA sequence according to claim 1 wherein the endothelin is human endothelin.
5. The DNA sequence according to claim 4, which has the DNA sequence shown in Fig. 4 or a portion thereof.
6. The DNA sequence according to claim 5 which has at least No. 261-No. 896 of the DNA sequence shown in Fig. 4.
7. The DNA sequence according to claim 5, which has No. 417-479 of the DNA sequence shown in Fig. 4.
8. A precursor of endothelin.
9. The precursor according to claim 8, which is a porcine endothelin precursor.
10. The precursor according to claim 9, whose amino acid sequence is shown in Fig. 2.
11. The precursor according to claim 8, which is a human endothelin precursor.
12. The precursor according to claim 11, whose amino acid sequence is shown in Fig. 4.
13. A transformant which is transformed by a DNA sequence comprising a DNA segment coding for endothelin.
14. The transformant according to claim 13, wherein the endothelin is porcine endothelin.
15. The transformant according to claim 14, wherein the DNA sequence of porcine endothelin is shown in Fig. 2 or a portion thereof.
16. The transformant according to claim 15, which is Esherichia coli XL-1/ppET4.4 (FERM P-9683)
17. The transformant according to claim 13, wherein the endothelin is human endothelin.
18. The transformant according to claim 17, wherein the DNA sequence of human endothelin is shown in Fig. 4 or a portion thereof.
19. The transformant according to claim 17 wherein the DNA sequence has at least No. 261-No. 896 of the DNA sequence shown in Fig. 4.
20. The transformant according to claim 17, which is Escherichia coli JM 103/pHET4-3(FERM P-9755), Saccharomyces cerevisiae AH 22R⁻/pGLD906-20 (FERM BP-1925), Escherichia coli N4830/pTS 4007 (FERM BP-2130), Escherichia coli DH1/pTS6003 (FERM BP-2131) or Saccharomyces cerevisiae AH 22R⁻ pGLD906-21 (FERM BP-2132).

21. A production method for mature endothelin or endothelin precursor which comprises culturing the transformant according to claim 13, accumulating mature endothelin or endothelin precussor in the culture medium and recovering the same.

22. The production method according to claim 21, wherein the transformant according to claim 14, 15, or 16 is used and the mature endothelin or endothelin precursor is porcine endothelin or porcine endothelin precussor.

23. The production method according to claim 21, wherein the transformant according to claim 17, 19 or 20 is used and te mature endothelin or endothelin precursor is human endothelin or human endothelin precussor.

24. A DNA sequence which comprises a sufficient number of the nucleotides shown in Figure 2 to produce a peptide whose mature product has a vasoconstrictive property.

25. A DNA sequence which comprises a sufficient number of the nucleotides shown in Figure 4 to produce a peptide whose mature product has a vasoconstrictive property.

26. A pharmaceutical composition which contains an effective amount of endothelin and pharmaceutically acceptable additional components.

27. A pharmaceutical composition according to claim 26, wherein the effective amount is 0.001 μg - 100 μg/kg.

28. A pharmaceutical composition according to claim 26 or 27, wherein the pharmaceutically acceptable additional components include vehicles, disintegrators, lubricants, binders, dispersants, fillers and carriers.

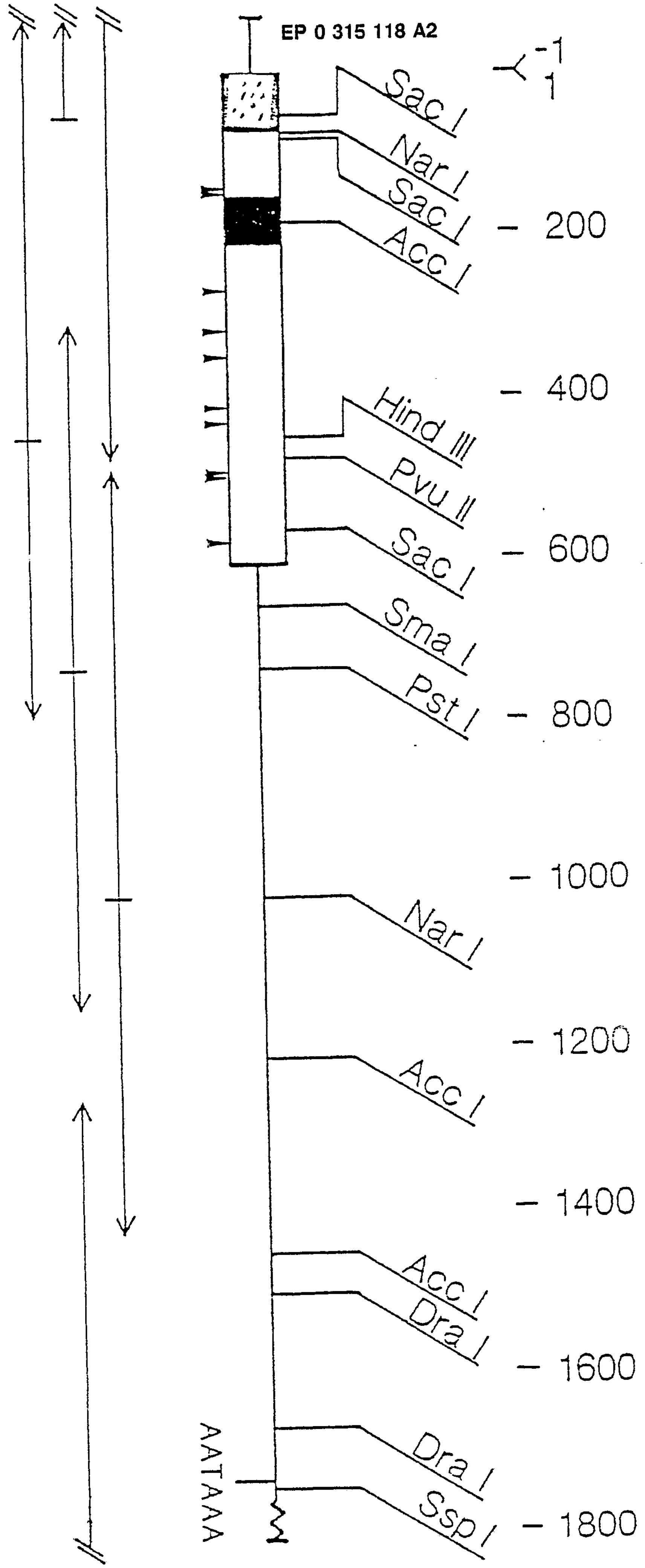

-1
1
Sac I
Nar I
Sac I
200
Acc I
400
Hind III
Pvu II
Sac I
600
Sma I
Pst I
800
1000
Nar I
1200
Acc I
1400
Acc I
Dra I
1600
Dra I
AATAAA
Ssp I
1800


FIG. 1

FIG. 2-1

```
-74   CATTTTTCTGCCTTTTTCCCCCCGCTTTCGGTTTAGTTCGCAGGGGGAGGATTTTGATCCTTTTTTTTTTTCAGA   -1

ATG GAT TAT TTC CCC ATG ATT ATC GCT CTG CTG TTT GTG GCT TTC CAA GGA GCT CCA GAA   60
Met Asp Tyr Phe Pro Met Ile Ile Ala Leu Leu Phe Val Ala Phe Gln Gly Ala Pro Glu   20

ACA GCG GTC CTG GGC GCC GAG CTC AGC CCG GAA GCC GAG AGC CAA GGG GAG ACG CCC TCT  120
Thr Ala Val Leu Gly Ala Glu Leu Ser Pro Glu Ala Glu Ser Gln Gly Glu Thr Pro Ser   40

CCC CAT GCA TCC TGG AGG CCC CGT CGG TCC AAG CGC TGC TCC TGC TCT TCC CTG ATG GAT  180
Pro His Ala Ser Trp Arg Pro Arg Arg Ser Lys Arg Cys Ser Cys Ser Ser Leu Met Asp   60

AAA GAG TGT GTC TAC TTC TGC CAC CTG GAC ATC ATC TGG GTC AAC ACT CCA GAA CAC ATT  240
Lys Glu Cys Val Tyr Phe Cys His Leu Asp Ile Ile Trp Val Asn Thr Pro Glu His Ile   80

GTC CCA TAC GGA CTT GGA AGC CCT TCT AGG TCC AGG CGA TCC TTA AAG GAT TTG TTT CCT  300
Val Pro Tyr Gly Leu Gly Ser Pro Ser Arg Ser Arg Arg Ser Leu Lys Asp Leu Phe Pro  100
```

FIG. 2-2

GCA AAG GCA GCA GAC CGC AGG GAT AGA TGC CAG TGT GCC AGC CAA AAA GAC AAG AAG TGC 360

Ala Lys Ala Ala Asp Arg Arg Asp Arg Cys Gln Cys Ala Ser Gln Lys Asp Lys Lys Cys 120

TGG AGT TTC TGC CAA GCA GGA AAA GAA ATC GGC AGG GAC CAA GAC ACA ATG GAG AAA CGC 420

Trp Ser Phe Cys Gln Ala Gly Lys Glu Ile Gly Arg Asp Gln Asp Thr Met Glu Lys Arg 140

TGG GAT AAC CAA AAG AAA GGA ACA GAC TGT TCC AAG CTT GGA GAG AAG TGT ATT CAT CGG 480

Trp Asp Asn Gln Lys Lys Gly Thr Asp Cys Ser Lys Leu Gly Glu Lys Cys Ile His Arg 160

CAG CTG GTG ATG GGA AGA AAA ATA AGA AGG TTG GAG GCC ATC AGC AAC AGC ATC AAA ACA 540

Gln Leu Val Met Gly Arg Lys Ile Arg Arg Leu Glu Ala Ile Ser Asn Ser Ile Lys Thr 180

TCT TTT CAC ATC GCC AAG CTG AAA GCC GAG CTC TAC AGA GAT AAG AAA GTG ACC CAT AAC 600

Ser Phe His Ile Ala Lys Leu Lys Ala Glu Leu Tyr Arg Asp Lys Lys Val Thr His Asn 200

CGA ACA CAC TGA TGACAGGTTGGCCGAGCCTGTCTGAAGCCATCTCGTCCCCAGGGAGCCCCGGGCCCGACTCTG 675

Arg Thr His *** 203

FIG. 2-3

CGCTCGCTTGGCAGGGGCTGAGATCAGAGCAGAAGCCTCCTCTGTTCAGACCGTTCCTTACTGCAGACTGGCACAGGAC 754
CAGCACCCTCGCCTGAACATTCCAGAAAAGGCTTAAGGAGTCCCCCCAGCCGGTCTTCATTGGCCTCCATCAGTGCTAA 833
GCGCGGCCGTCTGTCTCTCCTCCTTTGCGGTGATGATGGGCCACTCGAAGAAAGCAGAGAGAGACACGGATGCCACCTG 912
CAAACCAGGGTGGCCTCCTCCAGCCCAGAGGGAGGTGACGAGCTTCCACGTGCGGGCGGAGACCTCCAAGAGCGTCCGG 991
AGGGCGTGTTTTGTTGTCCCACGCAGGCGCCTGGCGCATTTCAGGGAGAAACACCCAAGTCCACACCAAGAGCTTTCCA 1070
AGGAAGGCGCAGACTGAAAAAACCCTCGAGGCACAAGCCCTCGAGTTAAAAATGAACAAGACTTTGCTTTCTTCCGTCT 1149
CGAGATGCAAAACCGAGAGAAACTGTTACTACTGGAAAATCAGGATGAGTTTCATGAATATGAGTCTACCTCACCTCTG 1228
CTGCACTCCGCCTAAAGTATTTCCTCACCTTTAATTATTTGCCTCCCCAAACTCCCCCCCCACCCCTCCCCCCAAGCCC 1307
CCACACTAAAATCCTAGCCTCGTGGAAATCTGTCTTACGTGTCAAGAGTAGATATAACACTTTCACGGTAATCTACTAG 1386
CTCTATTCCATAAGAAAAAAAATATCACTGAACCAGGAGATTCCCATGTCTTGATTTTTAGAAACACAAGGGTATACGG 1465
TTGTTGATGAGAGCTATTGAAGGGAAAACATTGGTTATGCTTTAAAGCAGTAAAATTATTTTCCTTTATGTAACTGGCA 1544
AGTGGAAGAGGTTGGATTAAATTTGGATGTACTTATTTTTTTACAGATATTTATATTCAAACAATTTATTCCTTATATT 1623
TACCATGTGAAATATATGTCTGGGCAGGCCATATTGGTCTATGTATTTTTTTAAAAAATGTATTTCTGAATGAAATTGA 1702
GAAAATGCTTCGTTTTGCCTATCAAGGTAATGACTTTAGAAAATAAATATTTTTTTTTCCCCT poly (A) 1764

FIG. 3

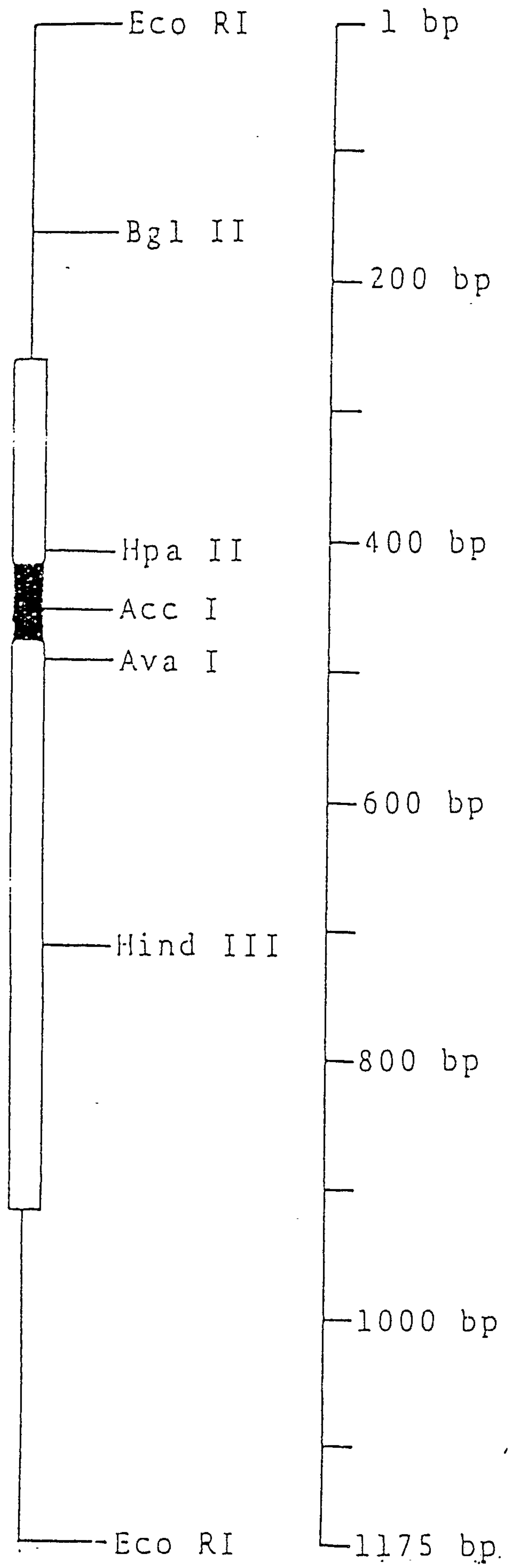
Eco RI
Bgl II
Hpa II
Acc I
Ava I
Hind III
Eco RI
1 bp
200 bp
400 bp
600 bp
800 bp
1000 bp
1175 bp

FIG. 4-1

```
      GA  ATT CGG GCT GCG CAG GCG AAC GGG TCC TGC GCC TCC TGC AGT CCC    47
 48  AGC TCT CCA CCG CCG CGT GCG CCT GCA GAC GCT CCG CTC GCT GCC TTC    95
 96  TCT CCT GGC AGG CGC TGC CTT TTC TCC CCG TTA AAG GGC ACT TGG GCT   143
144  GAA GGA TCG CTT TGA GAT CTG AGG AAC CCG CAG CGC TTT GAG GGA CCT   191
192  GAA GCT GTT TTT CTT CGT TTT CCT TTG GGT TCA GTT TGA ACG GGA GGT   239
240  TTT TGA TCC CTT TTT TTC AGA ATG GAT TAT TTG CTC ATG ATT TTC TCT   287
                             Met Asp Tyr Leu Leu Met Ile Phe Ser     9
288  CTG CTG TTT GTG GCT TGC CAA GGA GCT CCA GAA ACA GCA GTC TTA GGC   335
 10  Leu Leu Phe Val Ala Cys Gln Gly Ala Pro Glu Thr Ala Val Leu Gly    25
336  GCT GAG CTC AGC GCG GTG GGT GAG AAC GGC GGG GAG AAA CCC ACT CCC   383
 26  Ala Glu Leu Ser Ala Val Gly Glu Asn Gly Gly Glu Lys Pro Thr Pro    41
384  AGT CCA CCC TGG CGG CTC CGC CGG TCC AAG CGC TGC TCC TGC TCG TCC   431
 42  Ser Pro Pro Trp Arg Leu Arg Arg Ser Lys Arg Cys Ser Cys Ser Ser    57
432  CTG ATG GAT AAA GAG TGT GTC TAC TTC TGC CAC CTG GAC ATC ATT TGG   479
 58  Leu Met Asp Lys Glu Cys Val Tyr Phe Cys His Leu Asp Ile Ile Trp    73
480  GTC AAC ACT CCC GAG CAC GTT GTT CCG TAT GGA CTT GGA AGC CCT AGG   527
 74  Val Asn Thr Pro Glu His Val Val Pro Tyr Gly Leu Gly Ser Pro Arg    89
528  TCC AAG AGA GCC TTG GAG AAT TTA CTT CCC ACA AAG GCA ACA GAC CGT   575
 90  Ser Lys Arg Ala Leu Glu Asn Leu Leu Pro Thr Lys Ala Thr Asp Arg   105
576  GAG AAT AGA TGC CAA TGT GCT AGC CAA AAA GAC AAG AAG TGC TGG AAT   623
106  Glu Asn Arg Cys Gln Cys Ala Ser Gln Lys Asp Lys Lys Cys Trp Asn   121
624  TTT TGC CAA GCA GGA AAA GAA CTC AGG GCT GAA GAC ATT ATG GAG AAA   671
122  Phe Cys Gln Ala Gly Lys Glu Leu Arg Ala Glu Asp Ile Met Glu Lys   137
```

FIG. 4-2

```
 672  GAC TGG AAT AAT CAT AAG AAA GGA AAA GAC TGT TCC AAG CTT GGG AAA   719
 138  Asp Trp.Asn Asn His Lys Lys Gly Lys Asp Cys Ser Lys Leu Gly Lys   153

 720  AAG TGT ATT TAT CAG CAG TTA GTG AGA GGA AGA AAA ATC AGA AGA AGT   767
 154  Lys Cys Ile Tyr Gln Gln Leu Val Arg Gly Arg Lys Ile Arg Arg Ser   169

 768  TCA GAG GAA CAC CTA AGA CAA ACC AGG TCG GAG ACC ATG AGA AAC AGC   815
 170  Ser Glu Glu His Leu Arg Gln Thr Arg Ser Glu Thr Met Arg Asn Ser   185

 816  GTC AAA TCA TCT TTT CAT GAT CCC AAG CTG AAA GGC AAG CCC TCC AGA   863
 186  Val Lys Ser Ser Phe His Asp Pro Lys Leu Lys Gly Lys Pro Ser Arg   201

 864  GAG CGT TAT GTG ACC CAC AAC CGA GCA CAT TGG TGA CAG ACT TCG GGG   911
 202  Glu Arg Tyr Val Thr His Asn Arg Ala His Trp ***                   212

 912  CCT GTC TGA AGC CAT AGC CTC CAC GGA GAG CCC TGT GGC CGA CTC TGC   959
              ***

 960  ACT CTC CAC CCT GGC TGG GAT CAG AGC AGG AGC ATC CTC TGC TGG TTC  1007

1008  CTG ACT GGC AAA GGA CCA GCG TCC TCG TTC AAA ACA TTC CAA GAA AGG  1055

1056  TTA AGG AGT TCC CCC AAC CAT CTT CAC TGG CTT CCA TCA GTG GTA ACT  1103

1104  GCT TTG GTC TCT TCT TTC ATC TGG GGA TGA CAA TGG ACC TCT CAG CAG  1151
                                          ***

1152  AAA CAC ACA GTC ACA TTC GAA TTC
```

FIG. 5

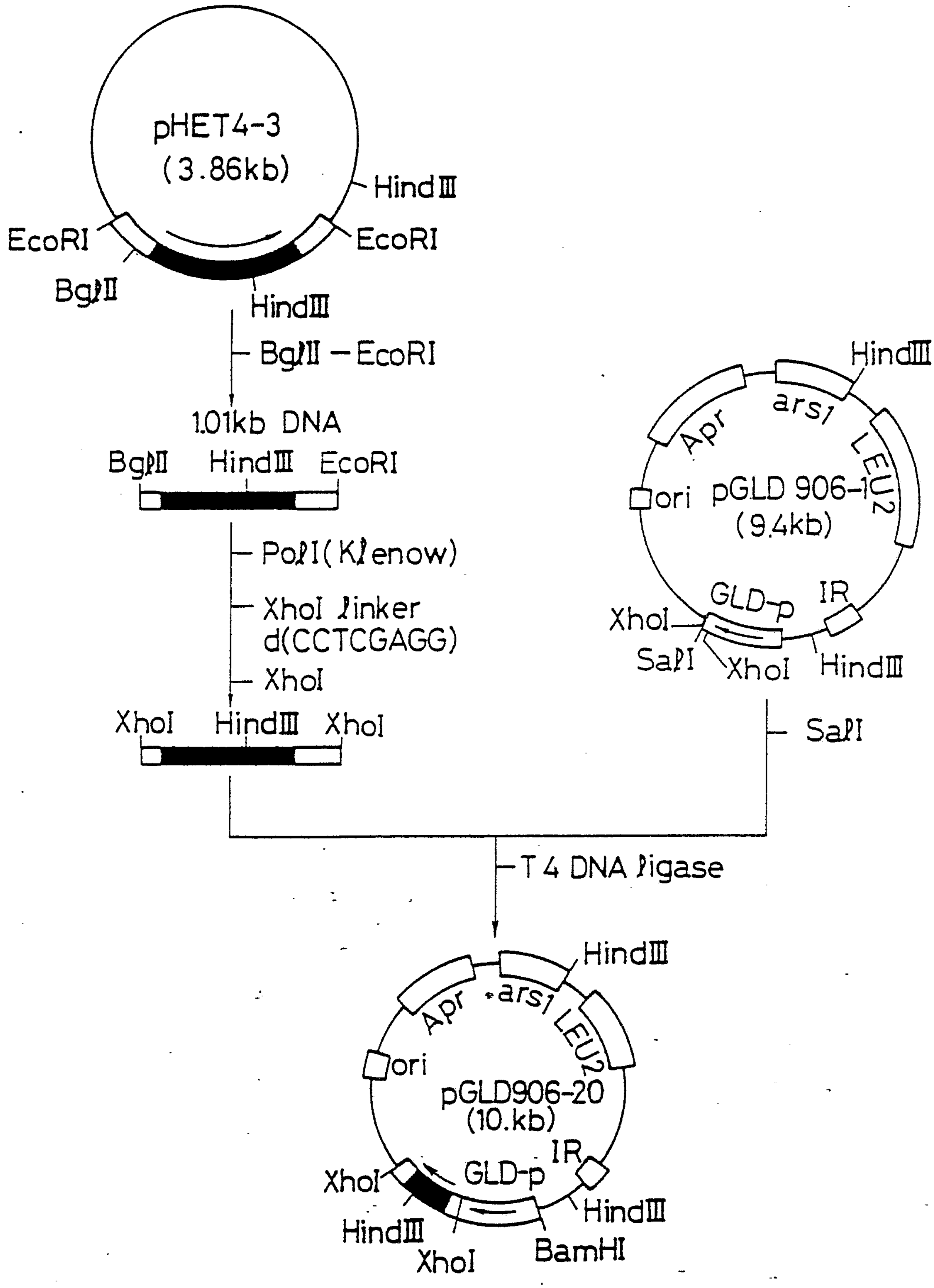
pHET4-3
(3.86kb)
HindIII
EcoRI
EcoRI
BglII
HindIII
BglII – EcoRI
1.01kb DNA
BglII HindIII EcoRI
PolI (Klenow)
XhoI linker
d(CCTCGAGG)
XhoI
XhoI HindIII XhoI
HindIII
ars1
Apr
LEU2
ori
pGLD 906-1
(9.4kb)
GLD-p
IR
XhoI
SalI
XhoI
HindIII
SalI
T4 DNA ligase
HindIII
Apr
ars1
LEU2
ori
pGLD906-20
(10.kb)
IR
GLD-p
XhoI
HindIII
HindIII
XhoI
BamHI

FIG. 6-1

```
H-260                                                              GAATTCGGGCTGCGCAGGCGAAC  -238
H GGGTCCTGCGCCTCCTGCAGTCCCAGCTCTCCACCGCCGCGTGCGCCTGCAGACGCTCCGCTCGCTGCCTTCTCTCCTG  -159
H GCAGGCGCTGCCTTTTCTCCCCGTTAAAGGGCACTTGGGCTGAAGGATCGCTTTGAGATCTGAGGAACCCGCAGCGCTT  -80

H TGAGGGACCTGAAGCTGTTTTTCTTCGTTTTCCTTTGGGTTCAGTTTGAACGGGAGGTTTTTGATCCCTTTTTTTCAGA  -1
P -74    CATTTTTCTGCCTTTTTCCCCCCGCTTTCGGTTTAGTTCGCAGGGGGAGGATTTTGATCCTTTTTTTTTTCAGA  -1

H ATG GAT TAT TTG CTC ATG ATT TTC TCT CTG CTG TTT GTG GCT TGC CAA GGA GCT CCA GAA  60
  Met Asp Tyr Leu Leu Met Ile Phe Ser Leu Leu Phe Val Ala Cys Gln Gly Ala Pro Glu  20
  Met Asp Tyr Phe Pro Met Ile Ile Ala Leu Leu Phe Val Ala Phe Gln Gly Ala Pro Glu  20
P ATG GAT TAT TTC CCC ATG ATT ATC GCT CTG CTG TTT GTG GCT TTC CAA GGA GCT CCA GAA  60

H ACA GCA GTC TTA GGC GCT GAG CTC AGC GCG GTG GGT GAG AAC GGC GGG GAG AAA CCC ACT  120
  Thr Ala Val Leu Gly Ala Glu Leu Ser Ala Val Gly Glu Asn Gly Gly Glu Lys Pro Thr  40
  Thr Ala Val Leu Gly Ala Glu Leu Ser Pro Glu Ala Glu Ser Gln Gly Glu Thr Pro Ser  40
P ACA GCG GTC CTG GGC GCC GAG CTC AGC CCG GAA GCC GAG AGC CAA GGG GAG ACG CCC TCT  120

H CCC AGT CCA CCC TGG CGG CTC CGC CGG TCC AAG CGC TGC TCC TGC TCG TCC CTG ATG GAT  180
  Pro Ser Pro Pro Trp Arg Leu Arg Arg Ser Lys Arg Cys Ser Cys Ser Ser Leu Met Asp  60
  Pro His Ala Ser Trp Arg Pro Arg Arg Ser Lys Arg Cys Ser Cys Ser Ser Leu Met Asp  60
P CCC CAT GCA TCC TGG AGG CCC CGT CGG TCC AAG CGC TGC TCC TGC TCT TCC CTG ATG GAT  180

H AAA GAG TGT GTC TAC TTC TGC CAC CTG GAC ATC ATT TGG GTC AAC ACT CCC GAG CAC GTT  240
  Lys Glu Cys Val Tyr Phe Cys His Leu Asp Ile Ile Trp Val Asn Thr Pro Glu His Val  80
  Lys Glu Cys Val Tyr Phe Cys His Leu Asp Ile Ile Trp Val Asn Thr Pro Glu His Ile  80
P AAA GAG TGT GTC TAC TTC TGC CAC CTG GAC ATC ATC TGG GTC AAC ACT CCA GAA CAC ATT  240
```

FIG. 6-2

```
H GTT CCG TAT GGA CTT GGA AGC CCT AGG TCC AAG AGA GCC TTG GAG AAT TTA CTT CCC ACA  300
  Val Pro Tyr Gly Leu Gly Ser Pro Arg Ser Lys Arg Ala Leu Glu Asn Leu Leu Pro Thr  100
  Val Pro Tyr Gly Leu Gly Ser Pro Ser Arg Ser Arg Arg Ser Leu Lys Asp Leu Phe Pro  100
P GTC CCA TAC GGA CTT GGA AGC CCT TCT AGG TCC AGG CGA TCC TTA AAG GAT TTG TTT CCT  300

H AAG GCA ACA GAC CGT GAG AAT AGA TGC CAA TGT GCT AGC CAA AAA GAC AAG AAG TGC TGG  360
  Lys Ala Thr Asp Arg Glu Asn Arg Cys Gln Cys Ala Ser Gln Lys Asp Lys Lys Cys Trp  120
  Ala Lys Ala Ala Asp Arg Arg Asp Arg Cys Gln Cys Ala Ser Gln Lys Asp Lys Lys Cys  120
P GCA AAG GCA GCA GAC CGC AGG GAT AGA TGC CAG TGT GCC AGC CAA AAA GAC AAG AAG TGC  360

H AAT TTT TGC CAA GCA GGA AAA GAA CTC AGG GCT GAA GAC ATT ATG GAG AAA GAC TGG AAT  420
  Asn Phe Cys Gln Ala Gly Lys Glu Leu Arg Ala Glu Asp Ile Met Glu Lys Asp Trp Asn  140
  Trp Ser Phe Cys Gln Ala Gly Lys Glu Ile Gly Arg Asp Gln Asp Thr Met Glu Lys Arg  140
P TGG AGT TTC TGC CAA GCA GGA AAA GAA ATC GGC AGG GAC CAA GAC ACA ATG GAG AAA CGC  420

H AAT CAT AAG AAA GGA AAA GAC TGT TCC AAG CTT GGG AAA AAG TGT ATT TAT CAG CAG TTA  480
  Asn His Lys Lys Gly Lys Asp Cys Ser Lys Leu Gly Lys Lys Cys Ile Tyr Gln Gln Leu  160
  Trp Asp Asn Gln Lys Lys Gly Thr Asp Cys Ser Lys Leu Gly Glu Lys Cys Ile His Arg  160
P TGG GAT AAC CAA AAG AAA GGA ACA GAC TGT TCC AAG CTT GGA GAG AAG TGT ATT CAT CGG  480

H GTG AGA GGA AGA AAA ATC AGA AGA AGT TCA GAG GAA CAC CTA AGA CAA ACC AGG TCG GAG  540
  Val Arg Gly Arg Lys Ile Arg Arg Ser Ser Glu Glu His Leu Arg Gln Thr Arg Ser Glu  180
  Gln Leu Val Met Gly Arg Lys Ile Arg Arg Leu Glu Ala Ile Ser Asn Ser Ile Lys Thr  180
P CAG CTG GTG ATG GGA AGA AAA ATA AGA AGG TTG GAG GCC ATC AGC AAC AGC ATC AAA ACA  540

H ACC ATG AGA AAC AGC GTC AAA TCA TCT TTT CAT GAT CCC AAG CTG AAA GGC AAG CCC TCC  600
  Thr Met Arg Asn Ser Val Lys Ser Ser Phe His Asp Pro Lys Leu Lys Gly Lys Pro Ser  200
  Ser Phe His Ile Ala Lys Leu Lys Ala Glu Leu Tyr Arg Asp Lys Lys Val Thr His Asn  200
P TCT TTT CAC ATC GCC AAG CTG AAA GCC GAG CTC TAC AGA GAT AAG AAA GTG ACC CAT AAC  600
```

FIG. 6-3

```
H AGA GAG CGT TAT GTG ACC CAC AAC CGA GCA CAT TGG TGA CAG ACT TCG GGG CCT GTC TGA   660
  Arg Glu Arg Tyr Val Thr His Asn Arg Ala His Trp ***                         ***   212
  Arg Thr His ***                                                                   203
P CGA ACA CAC TGA TGA CAG GTT GGC CGA GCC TGT CTG AAG CCA TCT CGT CCC CAG GGA GCC   660

H AGCCATAGCCTCCACGGAGAGCCCTGTGGCCGACTCTGCACTCTCCACCCTGGCTGGGATCAGAGCAGGAGCATCCTCT   739
P CCGGGCCCGACTCTGCGCTCGCTTGGCAGGGGCTGAGATCAGAGCAGAAGCCTCCTCTGTTCAGACCGTTCCTTACTGC   739

H GCTGGTTCCTGACTGGCAAAGGACCAGCGTCCTCGTTCAAAACATTCCAAGAAAGGTTAAGGAGTTCCCCCAACCATCT   818
P AGACTGGCACAGGACCAGCACCCTCGCCTGAACATTCCAGAAAAGGCTTAAGGAGTCCCCCCAGCCGGTCTTCATTGGC   818

H TCACTGGCTTCCATCAGTGGTAACTGCTTTGGTCTCTTCTTTCATCTGGGGATGACAATGGACCTCTCAGCAGAAACAC   897
P CTCCATCAGTGCTAAGCGCGGCCGTCTGTCTCTCCTCCTTTGCGGTGATGATGGGCCACTCGAAGAAAGCAGAGAGAGA   897

H ACAGTCACATTCGAATTC                                                                915
P CACGGATGCCACCTGCAAACCAGGGTGGCCTCCTCCAGCCCAGAGGGAGGTGACGAGCTTCCACGTGCGGGCGGAGACC   976

P TCCAAGAGCGTCCGGAGGGCGTGTTTTGTTGTCCCACGCAGGCGCCTGGCGCATTTCAGGGAGAAACACCCAAGTCCAC  1055
P ACCAAGAGCTTTCCAAGGAAGGCGCAGACTGAAAAAACCCTCGAGGCACAAGCCCTCGAGTTAAAAATGAACAAGACTT  1134
P TGCTTTCTTCCGTCTCGAGATGCAAAACCGAGAGAAACTGTTACTACTGGAAAATCAGGATGAGTTTCATGAATATGAG  1213
P TCTACCTCACCTCTGCTGCACTCCGCCTAAAGTATTTCCTCACCTTTAATTATTTGCCTCCCCAAACTCCCCCCCCACC  1292
P CCTCCCCCCAAGCCCCCACACTAAAATCCTAGCCTCGTGGAAATCTGTCTTACGTGTCAAGAGTAGATATAACACTTTC  1371
P ACGGTAATCTACTAGCTCTATTCCATAAGAAAAAAAATATCACTGAACCAGGAGATTCCCATGTCTTGATTTTTAGAAA  1450
P CACAAGGGTATACGGTTGTTGATGAGAGCTATTGAAGGGAAAACATTGGTTATGCTTTAAAGCAGTAAAATTATTTTCC  1529
P TTTATGTAACTGGCAAGTGGAAGAGGTTGGATTAAATTTGGATGTACTTATTTTTTTACAGATATTTATATTCAAACAA  1608
P TTTATTCCTTATATTTACCATGTGAAATATATGTCTGGGCAGGCCATATTGGTCTATGTATTTTTTTAAAAAATGTATT  1687
P TCTGAATGAAATTGAGAAAATGCTTCGTTTTGCCTATCAAGGTAATGACTTTAGAAAATAAATATTTTTTTTTCCCCTpoly(A)  1764
```

FIG.7

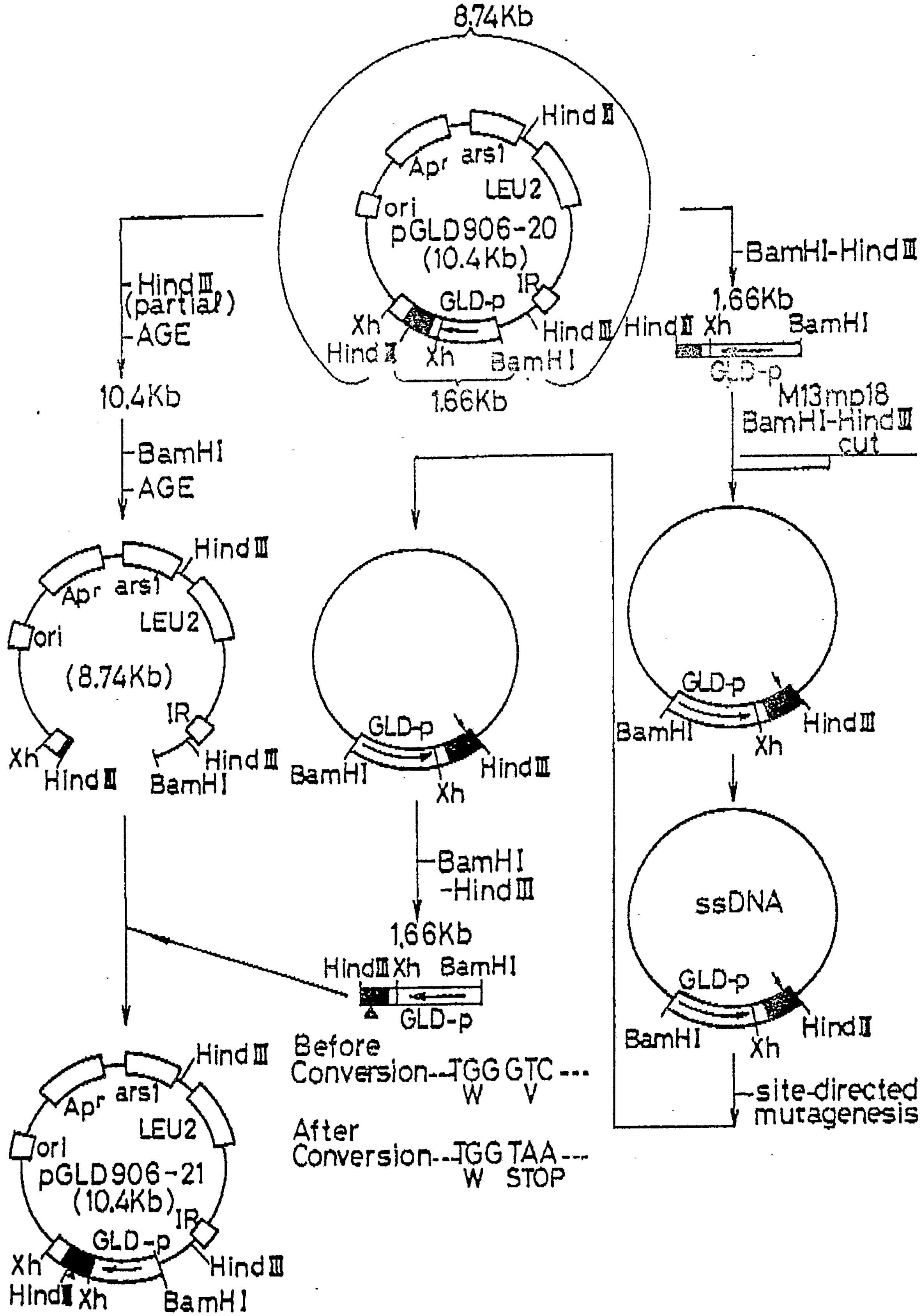
8.74Kb
Hind III
Apr
ars1
LEU2
ori
pGLD906-20
(10.4Kb)
GLD-p
IR
Xh
Hind III
Xh
BamHI
Hind III
1.66Kb
Hind III
(partial)
AGE
10.4Kb
BamHI
AGE
BamHI-Hind III
1.66Kb
Hind III Xh BamHI
GLD-p
M13mp18
BamHI-Hind III
cut
Hind III
Apr
ars1
LEU2
ori
(8.74Kb)
IR
Xh
Hind III
Hind III
BamHI
GLD-p
BamHI
Xh
Hind III
GLD-p
BamHI
Xh
Hind III
BamHI
-Hind III
1.66Kb
Hind III Xh BamHI
GLD-p
ssDNA
GLD-p
BamHI
Xh
Hind III
site-directed
mutagenesis
Before
Conversion---TGG GTC---
W V
After
Conversion---TGG TAA---
W STOP
Hind III
Apr
ars1
LEU2
ori
pGLD906-21
(10.4Kb)
IR
GLD-p
Xh
Hind III
Hind III
Xh
BamHI

FIG.8

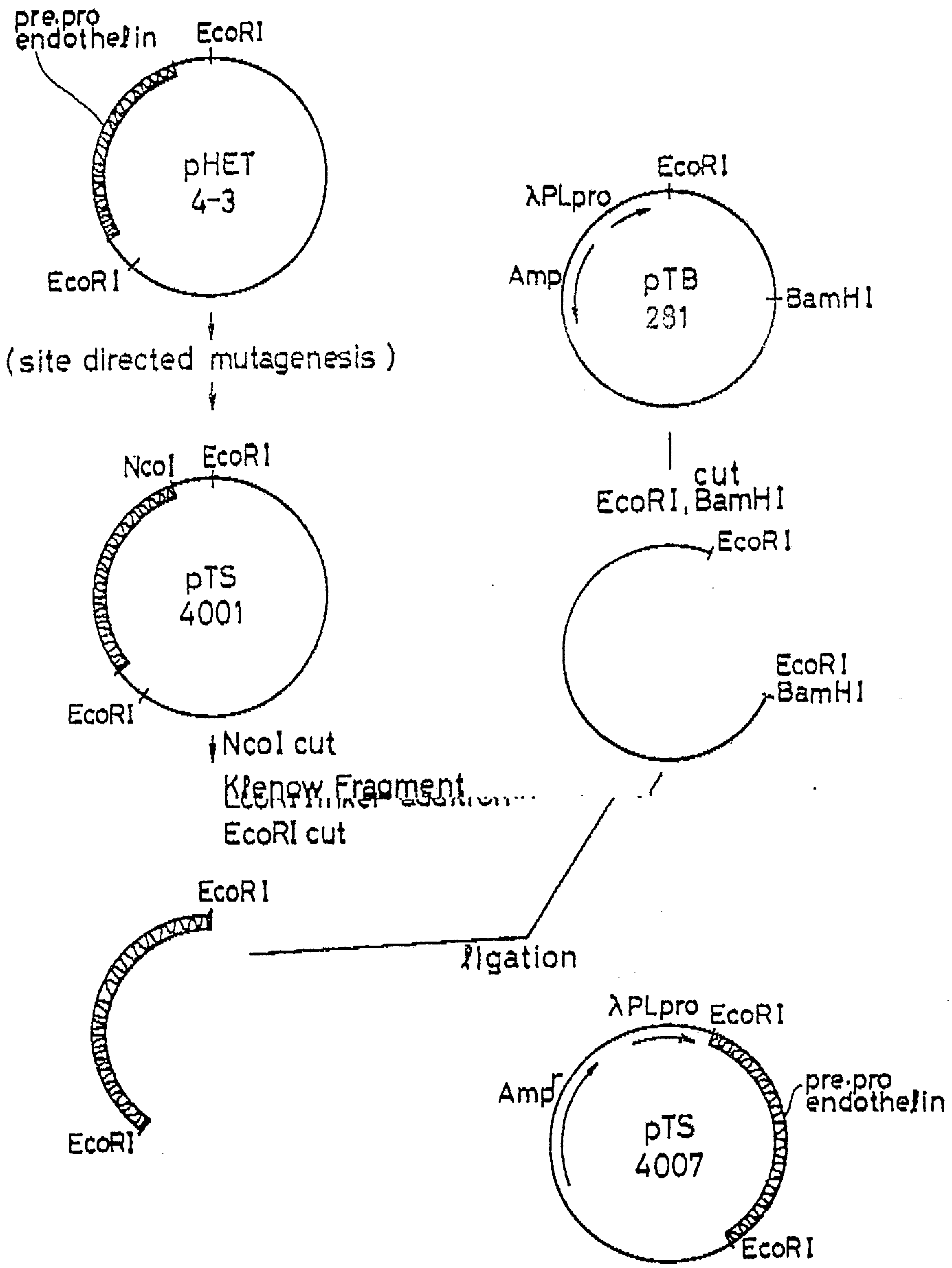
pre.pro endothelin
EcoRI
pHET 4-3
EcoRI
(site directed mutagenesis)
λPLpro
EcoRI
Amp
pTB 281
BamHI
cut EcoRI, BamHI
NcoI
EcoRI
pTS 4001
EcoRI
EcoRI
EcoRI BamHI
NcoI cut
Klenow Fragment
EcoRI linker addition
EcoRI cut
EcoRI
EcoRI
ligation
λPLpro
EcoRI
Amp
pTS 4007
pre.pro endothelin
EcoRI

FIG.9

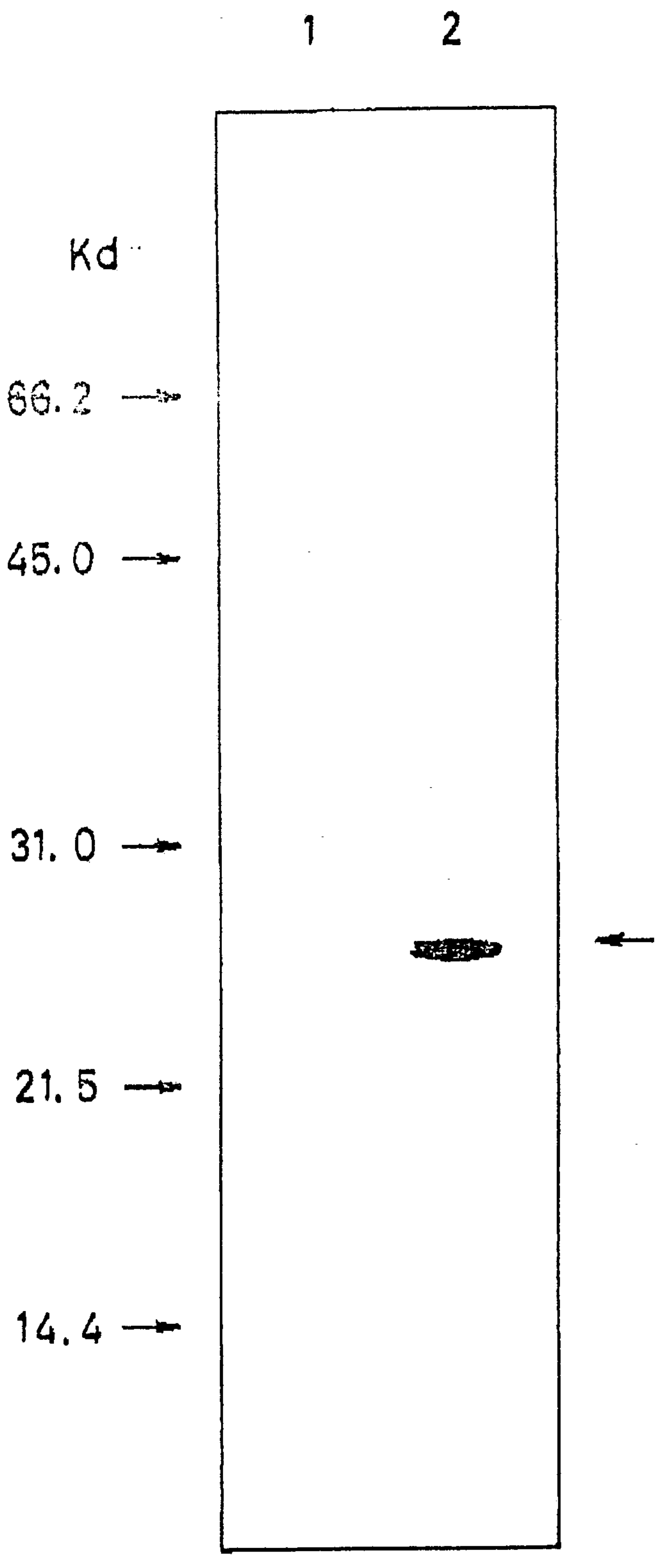

FIG.10

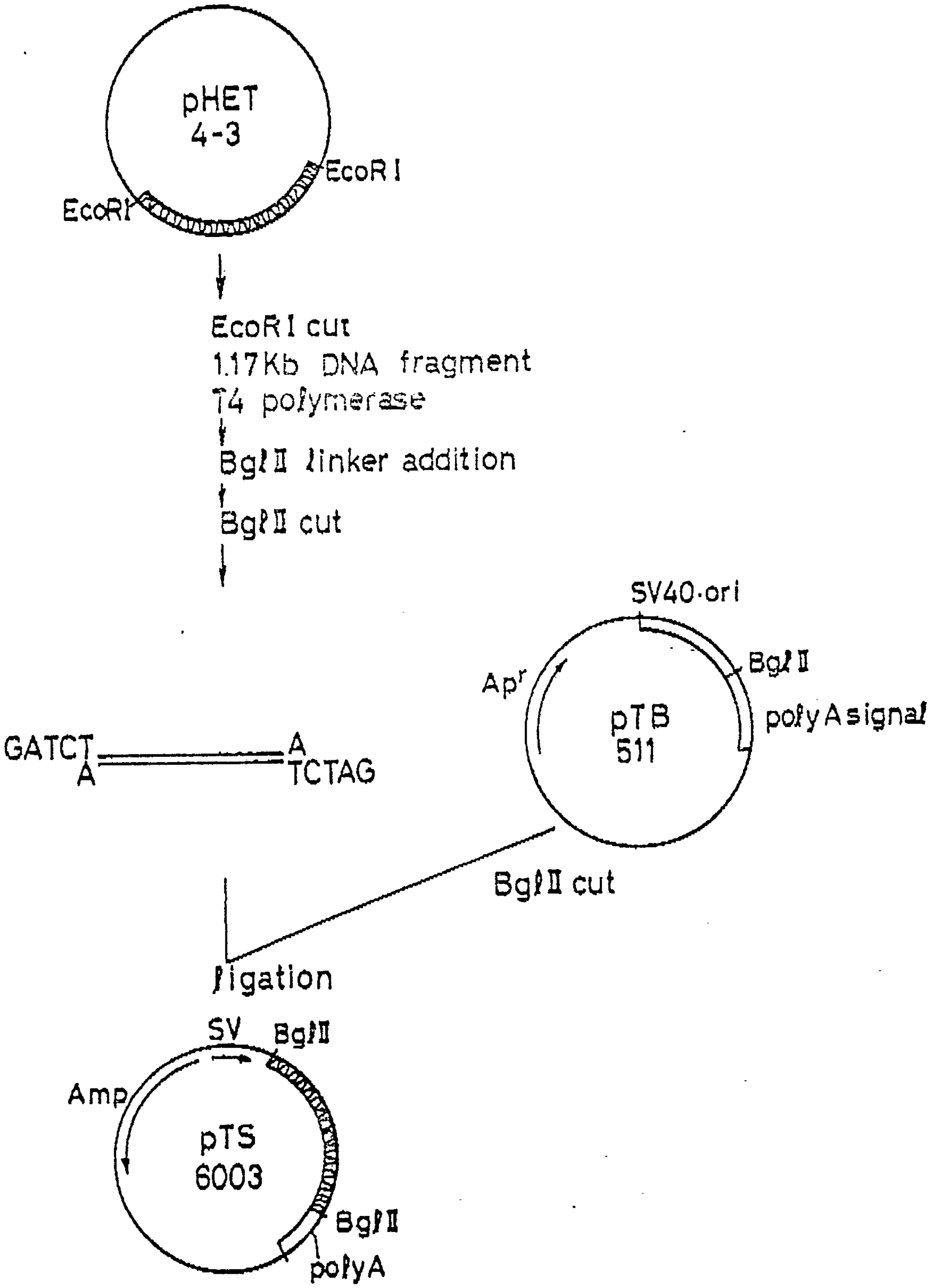
pHET
4-3
EcoRI
EcoRI
EcoRI cut
1.17Kb DNA fragment
T4 polymerase
BglII linker addition
BglII cut
GATCT
A
A
TCTAG
SV40·ori
BglII
Apr
pTB
511
polyA signal
BglII cut
ligation
SV
BglII
Amp
pTS
6003
BglII
polyA

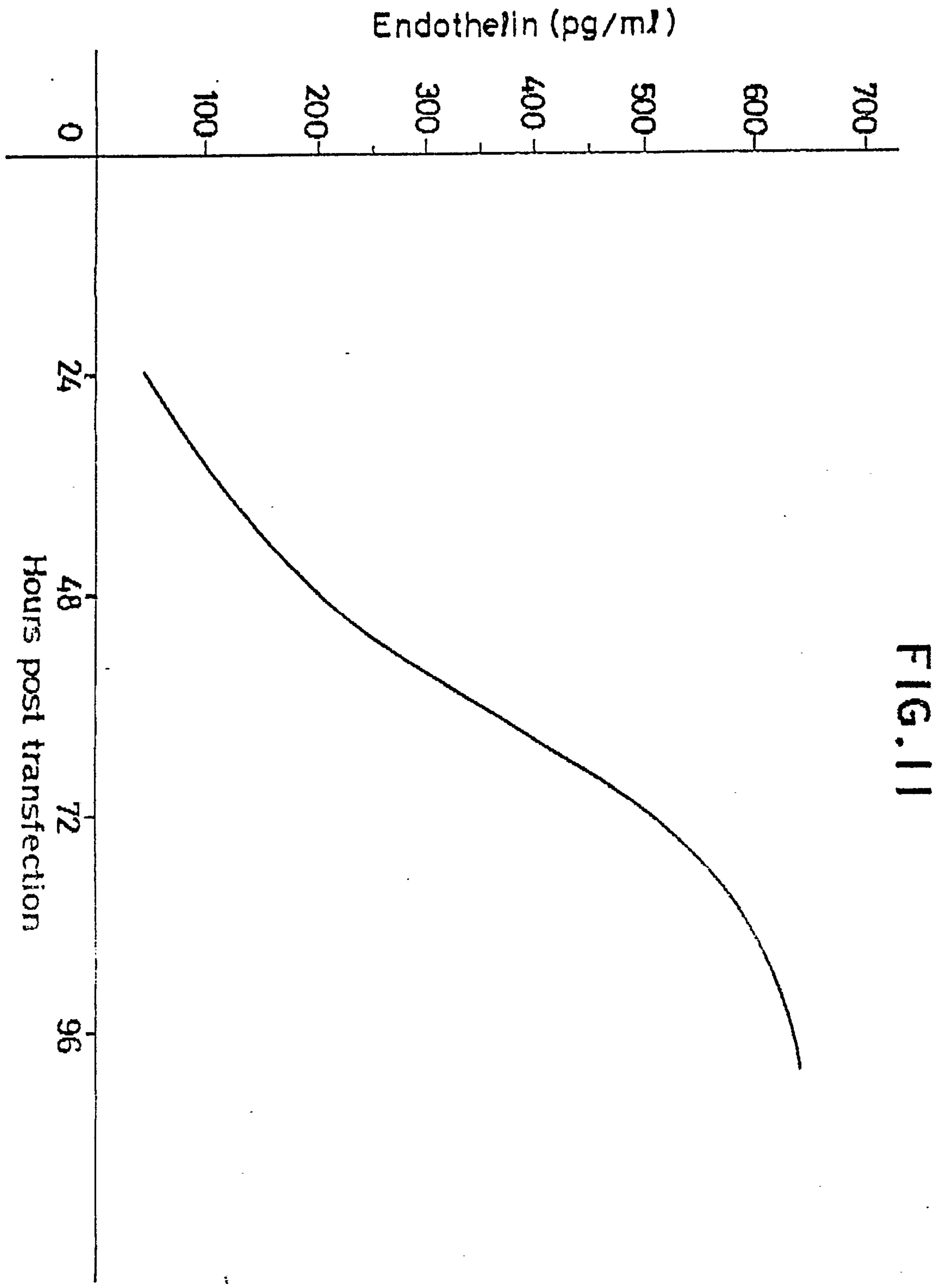
Endothelin (pg/ml)
0
100
200
300
400
500
600
700
24
48
72
96
Hours post transfection

FIG. 11